# EUROPEAN PATENT APPLICATION

(11) **EP 3 109 798 A1**
(43) Date of publication of application: **28.12.2016**
(21) Application number: 15201798.4
(22) Date of filing: 21.12.2015
(51) Int. Cl.: G06K 9/32, A61B 5/0205, A61B 5/024, A61B 5/08, A61B 5/1455, G06K 9/46, G06K 9/00

(54) **METHOD AND SYSTEM FOR DETERMINING EMOTIONS OF A USER USING A CAMERA**

(30) Priority: 27.06.2015 IN 3252CH2015; 14.08.2015 US 201514826815
(71) Applicant: Wipro Limited, 560 035 Karnataka (IN)
(72) Inventor: PANDIAN, Panneer Selvam Jayaveera, 600100 Tamil Nadu (IN); PATHANGAY, Vinod, 560100 Bangalore (IN)
(74) Representative: Finnegan Europe LLP

(57) **Abstract**

The present disclosure relates to a method for determining emotions of a user using a camera. The method comprises receiving at least one image of the user from the camera. Then, at least one region of interest of the user is detected in the at least one image. A video plethysmographic waveform is generated by analyzing the at least one region of interest. Then, at least one physiological characteristic based on the video plethysmographic waveform is determined. The emotions of the user are determined by comparing the at least one physiological characteristic with predefined physiological characteristics defined for each emotion.

## Description

### FIELD OF THE DISCLOSURE

The present subject matter is related, in general to human behavior detection, and more particularly, but not exclusively to an emotion detection system and method for determining emotions of a user using a camera.

### BACKGROUND

An individual may undergo various emotions, such as anger, anxiety, happiness, sadness, fear, anger, surprise and disgust. Nowadays, sensing and understanding the individual's emotions have become crucial for one or more scenarios of society. Considering a business scenario, where sensing and understanding emotions of a customer while dealing with the customer helps in offering the right products or services to the customer. In such a way, in any commercial business transactions, the customer and a service provider can be benefitted in an effective manner. In a scenario of public breach like dacoity, terrorism etc., sensing and understanding of the individual's emotions desiring to cause such public breach is significant to stop the individual from committing such public breach. Other scenarios can include interviewing candidate, where an interviewer can ask a particular level and type of questions as per the emotions of the candidate.

In one conventional method, an individual's emotions may be detected based on facial expressions of the individual. The facial expressions are generally observed through video feeds captured using a camera. However, such a way of determining emotions of the individual from the facial expressions is error prone because the individual can manipulate the facial expression for suppressing actual emotions, mood and intent of the individual. Hence, such a conventional method of determining the emotions from the facial expressions may not indicate the actual emotions of the individual.

In another conventional method, an individual's physiological parameters may be measured using wearable devices, that is, the wearable devices are placed on the individual to measure the physiological parameters. However, such wearable devices are expensive and the user may not be able to afford such devices or may not be willing to spend extra on the wearable devices. Further, measurement of the physiological parameters using the wearable devices may not help to determine the emotions accurately of the individual since some physiological parameters may get erroneously captured by the wearable devices.

### SUMMARY

One or more shortcomings of the prior art are overcome and additional advantages are provided through the present disclosure. Additional features and advantages are realized through the techniques of the present disclosure. Other embodiments and aspects of the disclosure are described in detail herein and are considered a part of the claimed disclosure.

In one embodiment, the present disclosure relates to a method for determining emotions of a user using a camera. The method comprises receiving at least one image of the user from the camera. The method further comprises detecting at least one region of interest of the user in the at least one image. The method further comprises generating a video plethysmographic waveform by analyzing the at least one region of interest. The method further comprises determining at least one physiological characteristic based on the video plethysmographic waveform. The method further comprises determining the emotions of the user by comparing the at least one physiological characteristic with predefined physiological characteristics defined for each emotion.

In another embodiment, the present disclosure relates to an emotion detection system for determining emotions of a user using a camera. The system further comprises a processor and a memory communicatively coupled to the processor, wherein the memory stores processor-executable instructions, which, on execution, cause the processor to perform operations comprising receiving at least one image of the user from the camera. The operations further comprise detecting at least one region of interest of the user in the at least one image. The operations further comprise generating a video plethysmographic waveform by analyzing the at least one region of interest. The operations further comprise determining at least one physiological characteristic based on the video plethysmographic waveform. The operations further comprise determining the emotions of the user by comparing the at least one physiological characteristic with predefined physiological characteristics defined for each emotion.

In another embodiment, the present disclosure relates to a non-transitory computer readable medium including instructions stored thereon that when processed by at least one processor causes an emotion detection system to perform the act of receiving at least one image of the user from the camera. The act further comprises detecting at least one region of interest of the user in the at least one image. The act further comprises generating a video plethysmographic waveform by analyzing the at least one region of interest. The act further comprises determining at least one physiological characteristic based on the video plethysmographic waveform. The act further comprises determining emotions of the user by comparing the at least one physiological characteristic with predefined physiological characteristics defined for each emotion.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF THE DRAWINGS

The accompanying drawings, which are incorporated in and constitute a part of this disclosure, illustrate exemplary embodiments and, together with the description, serve to explain the disclosed principles. In the figures, the left-most digit(s) of a reference number identifies the figure in which the reference number first appears. The same numbers are used throughout the figures to reference like features and components. Some embodiments of system and/or methods in accordance with embodiments of the present subject matter are now described, by way of example only, and with reference to the accompanying figures, in which:
**Figure 1** illustrates an exemplary embodiment of generation of Video Plethysmography Waveforms (VPW) of Region of Interest (ROI) of a user to determine emotions of the user in accordance with some embodiments of the present disclosure;
**Figure 2** illustrates an exemplary embodiment of environment for determining emotions of a user using Video Plethysmographic Waveforms (VPW) in accordance with some embodiments of the present disclosure;
**Figure 3** illustrates a block diagram of an exemplary emotion detection system with various data and modules for determining emotions of a user in accordance with some embodiments of the present disclosure;
**Figure 4** shows the Video Plethysmographic Waveforms (VPW) being generated for the at least one region of interest in accordance with some embodiments of the present disclosure;
**Figure 5** shows a flowchart illustrating a method for determining emotions of a user using a camera in accordance with some embodiments of the present disclosure; and
**Figure 6** is a block diagram of an exemplary computer system for implementing embodiments consistent with the present disclosure.

It should be appreciated by those skilled in the art that any block diagrams herein represent conceptual views of illustrative systems embodying the principles of the present subject matter. Similarly, it will be appreciated that any flow charts, flow diagrams, state transition diagrams, pseudo code, and the like represent various processes which may be substantially represented in computer readable medium and executed by a computer or processor, whether or not such computer or processor is explicitly shown.

### DETAILED DESCRIPTION

In the present document, the word "exemplary" is used herein to mean "serving as an example, instance, or illustration." Any embodiment or implementation of the present subject matter described herein as "exemplary" is not necessarily to be construed as preferred or advantageous over other embodiments.

While the disclosure is susceptible to various modifications and alternative forms, specific embodiment thereof has been shown by way of example in the drawings and will be described in detail below. It should be understood, however that it is not intended to limit the disclosure to the particular forms disclosed, but on the contrary, the disclosure is to cover all modifications, equivalents, and alternative falling within the scope of the disclosure.

The terms "comprises", "comprising", or any other variations thereof, are intended to cover a non-exclusive inclusion, such that a setup, device or method that comprises a list of components or steps does not include only those components or steps but may include other components or steps not expressly listed or inherent to such setup or device or method. In other words, one or more elements in a system or apparatus proceeded by "comprises... a" does not, without more constraints, preclude the existence of other elements or additional elements in the system or apparatus.

In the following detailed description of the embodiments of the disclosure, reference is made to the accompanying drawings that form a part hereof, and in which are shown by way of illustration specific embodiments in which the disclosure may be practiced. These embodiments are described in sufficient detail to enable those skilled in the art to practice the disclosure, and it is to be understood that other embodiments may be utilized and that changes may be made without departing from the scope of the present disclosure. The following description is, therefore, not to be taken in a limiting sense.

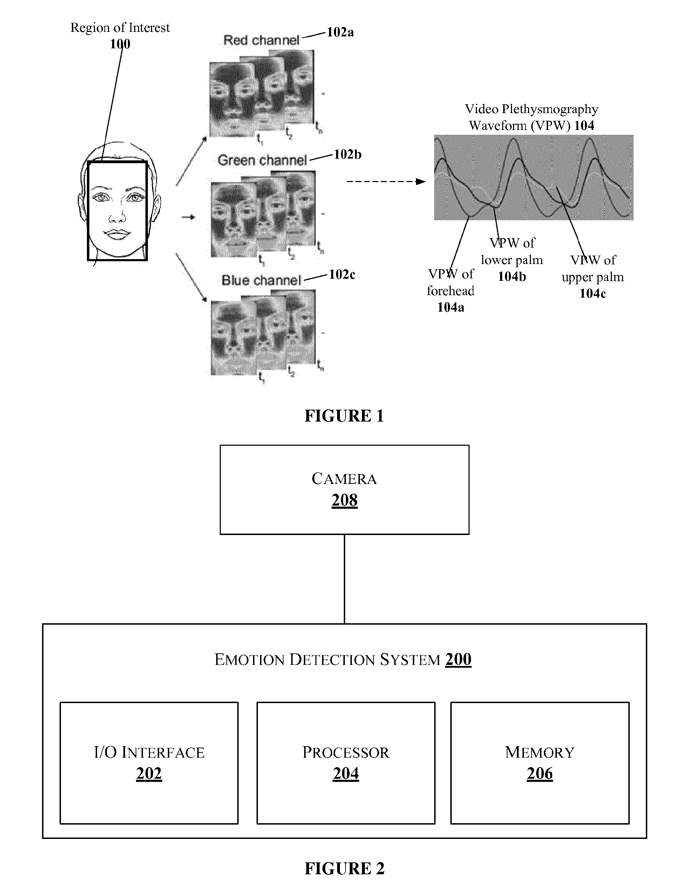

The present disclosure relates to a method and an emotion detection system for determining emotions of a user using a camera. The method comprises receiving at least one image of the user from the camera which is communicatively connected to the emotion detection system. In an embodiment, the at least one image is a series of images taken from a video frame. In an embodiment, the camera is configured in the emotion detection system and/or coupled to the emotion detection system. In an embodiment, the camera can be connected to the emotion detection system over a network comprising wired or wireless network. From the received at least one image of the user, regions of interest of the user is detected. More particularly, the regions of interest are the uncovered body parts of the user. Then, Video Plethysmographic Waveforms (VPW) are generated by analyzing the regions of the interest. Plethysmography is a measure of variations in a body part resulting from changes in the amount of blood passing through it. Particularly, the video plethysmographic waveforms are generated based on pixel variations of the at least one image of the user corresponding to the detected regions of the interest. **Figure 1** shows VPW **104** generated from the region of interest **100** of the user. Consider the regions of interest **100** are face, upper palm of the user and lower palm of the user. The VPW **104** is generated from three different channels comprising Red channel **102a,** Green Channel **102b** and Blue Channel **102c,** which are formed from the regions of interest **100** of the at least one image. For each channel, VPW **104** is generated. In the illustrated **figure 1****,** VPW **104** is generated for each regions of the interest based on the corresponding channel. That is, VPW **104** is generated for the face, the upper palm and the lower palm region of the user. Based on the video plethysmographic waveforms, at least one physiological characteristic including, but not limiting to, Peripheral capillary Oxygen Saturation (SPO2), respiratory rate and heart rate of the user is determined. The at least one physiological characteristic is compared with predefined physiological characteristics defined for each emotion. Based on the comparison, the emotions of the user such as happiness, sadness, fear anger, surprise and disgust etc. are determined. In an embodiment, the predefined physiological characteristics defined for the each emotion are updated based on a feedback received on the emotions determined for the user. In such a way, the emotions of the user from the physiological characteristics of the user determine the actual, accurate and correct emotions of the user.

**Figure 2** illustrates an exemplary embodiment of environment for determining emotions of a user using Video Plethysmographic Waveforms (VPW) in accordance with some embodiments of the present disclosure.

In one implementation, the emotion detection system **200** may be implemented in a variety of computing systems, such as a laptop computer, a desktop computer, a Personal Computer (PC), a notebook, a smartphone, a tablet, e-book readers (e.g., Kindles and Nooks), a server, a network server, and the like. In one example, the emotion detection system **200** is configured to determine emotions of the user non-invasively. Particularly, the emotions of the user are detectable from video frames captured. Thus, any wearable device and/or contact of the user with the emotion detection system **200** and/or any device connected to the emotion detection system **200** is excluded. The components of the emotion detection system **200** are explained in detail below sections of the description.

In an embodiment, the emotion detection system **200** is communicatively connected to at least one camera **208.** In one example, the at least one camera **208** includes, but is not limited to, a video camera, digital camera, Charged Couple Device (CCD) camera, an image camera, Universal Serial Bus (USB) camera, video cards with composite or S-video devices and other such camera which is capable of capturing video frames of users. Here, the users are persons or subjects whose at least one image is captured and emotions are to be determined. In one implementation, the at least one camera **208** is a separate device which is coupled to the emotion detection system **200** and/or connected to the emotion detection system **200** over a network. In one implementation, the at least one camera **208** is configured in one or more user devices (not shown) used by the users. The one or more user devices include, but are not limited to, computing systems, such as a laptop computer, a desktop computer, a Personal Computer (PC), a notebook, a smartphone, a smartwatch, a wearable device, a tablet, e-book readers (e.g., Kindles and Nooks). In such a case, the emotion detection system **200** may be communicatively connected to the one or more user devices and one or more servers (not shown). In one implementation, the at least one image of the user can be received from the one or more user devices and/or the one or more servers (not shown) by the emotion detection system **200.** In such a case, the at least one image of the user may be stored in files/library of the one or more user devices, and/or memory chip, USBs, hard disks, and/or the one or more servers. Here, the one or more servers are a server associated with the emotion detection system **200** and/or third party servers accessible by the emotion detection system **200.** In one implementation, the at least one image of the user can be downloaded from Internet.

In the illustrated **figure 2****,** the emotion detection **200** comprises an I/O interface **202,** a central processing unit ("CPU" or "processor") **204** having one or more processing units, and a memory **206** in accordance with some embodiments of the present disclosure.

The I/O interface **202** is a medium through which the at least one image of the user can be received from the at least one camera **208,** and/or the one or more user devices and/or the one or more servers. Further, the I/O interface **202** is configured to receive a feedback from the users and/or operators who are capable of operating the emotion detection system **200.** The I/O interface **202** provides results on determining the emotions of the user. Particularly, the emotions of the user are provided to a display unit (not shown in **figure 1**), and the one or more user devices. The I/O interface **402** is coupled with the processor **404.**

The processor **204** may comprise at least one data processor for executing program components for processing system-generated video plethysmographic waveforms of corresponding regions of interest of the user from the at least one image of the user. The processor **204** is configured to detect at least one region of interest of the user. In an exemplary embodiment, the region of interest of the user is uncovered body part of the user. The processor **202** analyzes the at least one region of interest of the user and forms a colored histogram i.e. a red channel, and/or a green channel and/or a blue channel of the at least region of interest. In an embodiment, only the green channel of the at least one region of interest is analyzed. Then, the processor **202** generates Video Plethysmographic Waveforms (VPW) for the colored histogram of the at least one region of interest of the user. The processor **202** generates the VPW based on pixel variations of the at least one image of the user of the region of interest. The processor **202** determines at least one physiological characteristic of the user based one the VPW. The processor **202** compares the at least one physiological characteristic of the user with predefined physiological characteristics defined for each emotion. The processor **202** is configured to determine the emotions of the user based on the comparison. In an embodiment, the processor **202** is configured to process the feedback received from the user and/or the operator for updating the predefined physiological characteristics.

The memory **206** stores instructions which are executable by the at least one processor **204.** In an embodiment, the memory **206** stores image information, region of interest data, VPW data, physiological characteristic data, predefined physiological data and an emotion list. In an embodiment, the image information, the region of interest data, the VPW data, the physiological characteristic data, the predefined physiological data and the emotion list are stored as one or more data required for determining the emotions of the user as described in the following description of the disclosure.

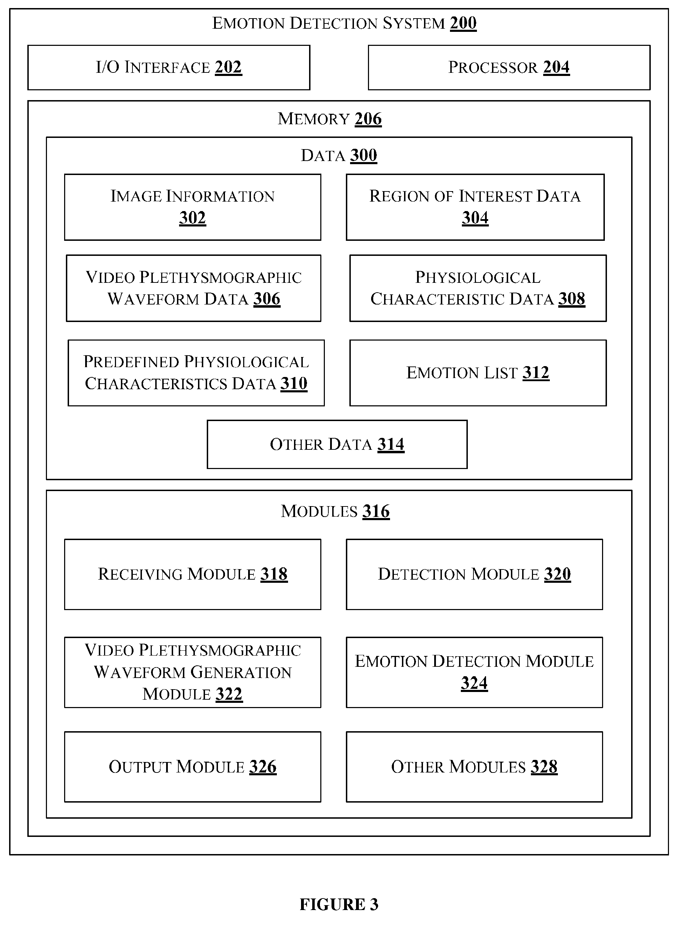

**Figure 3** illustrates a block diagram of the exemplary emotion detection system **200** with various data and modules for determining the emotions of the user in accordance with some embodiments of the present disclosure. In the illustrated **figure 3****,** the one or more data **300** and the one or more modules **316** stored in the memory **206** are described herein in detail.

In an embodiment, the one or more data **300** may include, for example, the image information **302,** the region of interest data **304,** the VPW data **306,** the physiological characteristic data **308,** the predefined physiological data **310** and the emotion list **312,** and other data **314** for determining the emotions of the user. In an embodiment, the data **300** including the image information **302,** the region of interest data **304,** the VPW data **306,** the physiological characteristic data **308** are the data which are detected and/or calculated in real-time. Particularly, the image information **302,** the region of interest data **304,** the VPW data **306,** the physiological characteristic data **308** are not predefined or preconfigured beforehand in the emotion detection system **200.** The predefined physiological data **310** and the emotion list **312** are defined beforehand and stored in the emotion detection system **200.**

The image information **302** refers to information of pixels of the at least one image of the user. The information of the pixels of the at least image may include, but is not limiting to, pixel size, pixel color and number of pixels of the at least one image.

The region of interest data **304** refers to the at least one region of interest of the user. For example, face and hands may be the at least one region of interest of the user.

The VPW data **306** refers to the VPW of the corresponding at least one region of interest, which is being generated based on pixel variations of the at least one image of the user. For instance, the VPW may be generated based on pixel variations observed between more than one, or moreover a plurality, of the images of the user. Such observations of the plurality of images may be carried out over a predetermined time period. The VPW data **306** includes details of the VPW including depth, width, altitude, distortion/noise of the waveforms being generated.

The physiological characteristic data **308** refers to the at least one physiological characteristic being determined from the VPW. The physiological characteristic data **308** may include, but is not limited to, SPO2, respiratory rate and heart rate of the user, which are being measured from the corresponding generated VPW.

The predefined physiological characteristics data **310** includes the physiological characteristics which includes, but is not limited to, SPO2, respiratory rate and heart rate that are defined for each corresponding emotion during configuration. In an example, the predefined physiological characteristics data 310 may also comprise at least one of training sequence waveforms. The training sequence waveforms are implemented using machine learning techniques and correspond to various emotions the user may undergo. The training sequence waveforms may be used for identifying emotions by comparing with the VPW.

The emotion list **312** refers to list of all the emotions of user in general. The emotion list **312** may include, but not limited to, happiness, sadness, fear, anger, surprise and disgust and other emotions of the user may be exhibited in a human being. In an embodiment, the predefined physiological characteristics data **310** and the emotion list **312** may be charted together in the memory **206.** Particularly, for each physiological characteristics data **310,** a corresponding emotion is defined and stored.

The other data **314** may refer to such data which can be referred for determining the emotions of the user.

In an embodiment, the one or more data **300** in the memory **206** are processed by the one or more modules **316** of the emotion detection system **200.** The one or more modules **316** may be stored within the memory **206** as shown in **Figure 3****.** In an example, the one or more modules **316,** communicatively coupled to the processor **204,** may also be present outside the memory **206** and implemented as hardware. As used herein, the term module refers to an application specific integrated circuit (ASIC), an electronic circuit, a processor (shared, dedicated, or group) and memory that execute one or more software or firmware programs, a combinational logic circuit, and/or other suitable components that provide the described functionality.

In one implementation, the one or more modules **316** may include, for example, a receiving module **318,** a detection module **320,** a VPW generation module **322,** an emotion detection module **324,** and an output module **326.** The memory **206** may also comprise other modules **328** to perform various miscellaneous functionalities of the emotion detection system **200.** It will be appreciated that such aforementioned modules may be represented as a single module or a combination of different modules.

The receiving module **318** receives the at least one image of the user from the at least one camera **208** and/or the one or more user devices and/or the one or more servers. For example, consider the camera **208** in a public place such as railway station, shopping malls, bus terminus, highways etc. In such a case, the at least one image of each of the users or persons is captured by the camera which is in turn received by the receiving module **318.**

The detection module **320** detects the at least one region of interest of the user from the received at least one image of the user. In an embodiment, the detection module **320** detects the at least one region of interest which is body part of the user that is not covered. For example, the detection module **320** can detect face, lower palm and upper palm as the region of interest from the at least one image of the user.

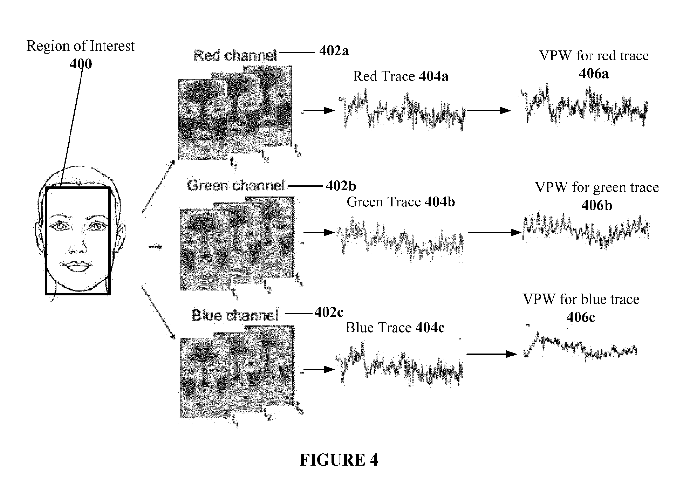

The Video Plethysmographic Waveform (VPW) generation module **322** analyzes the at least one region of interest of the user being detected. The VPW generation module **322** generates a color histogram i.e. video plethysmographic gradient histogram of the at least one region of interest. In an embodiment, the color histogram includes, but is not limited to, red channel, green channel and blue channel. Then, the waveforms of the at least one region of interest is generated from the color histogram in a form of corresponding trace, for example, red trace, green trace and blue trace. Then, the VPW generation module **322** generates the VPW of the corresponding trace. In an example, since emotions of the users are dynamic and may change over a short time, the VPW generation module **322** may generate the VPW by monitoring the at least one regions of interest for a predefined time period. For instance, the VPW generation module **322** may monitor the region of interest and receive video feed of the region of interest for 30 seconds. Thereafter, the VPW generation module **322** may generate the VPW for the 30 seconds to identify the emotion of the user. **Figure 4** shows the VPW being generated for the at least one region of interest **400.** For example, the image of the user is captured, a plurality of times, or more specifically for 'n' number of times i.e. 'n' number of frames per second i.e. t1, t2,...,tn of the image of the user is received. Consider, the region of interest **400** is face. Then, the color histogram of the face is generated with red channel **402a,** with green channel **402b** and blue channel **402c** for each of the frames. Then, for each color histogram, the waveforms in the form of the traces having red trace **404a,** green trace **404b** and blue trace **404c** is formed. Then, for each trace, VPW **406a, 406b and 406c** is generated as shown in **figure 4****.** In an embodiment, the VPW is generated based on the pixel variations of the at least one image, corresponding to each of the at least one region of interest.

The emotion detection module **324** determines at least one physiological characteristic of the user based on the VPW being generated. The at least one physiological characteristic includes, but is not limited to SPO2, the respiratory rate and the heart rate of the user. Then, the emotion detection module **324** compares the at least one physiological characteristic with the predefined physiological characteristics. If the at least one physiological characteristic matches with the predefined physiological characteristic, then the emotion corresponding to the predefined physiological characteristic is determined for the user. In one implementation, the emotion detection module **324** may compare the VPW generated with the at least one of training sequence waveforms to identify the emotion the user is going through. In an example, upon determining the emotion by comparing the VPW and the at least one of training sequence waveforms, the emotion detection module **324** may validate the emotion of the user by matching the at least one physiological characteristic with the predefined physiological characteristics. In case the emotion detection module **324** identifies that the at least one physiological characteristic doesn't match with the predefined physiological characteristic, the emotion detection module **324** may use the at least one physiological characteristic and the emotion identified for training the emotion detection system **200.**

The output module **326** provides the detected emotions of the user to at least one of the display unit, the one or more user devices and the one or more servers. In an embodiment, the feedback from the user is received from the user by the receiving module **318** and the predefined physiological characteristics is updated by the output module **326** based on the feedback.

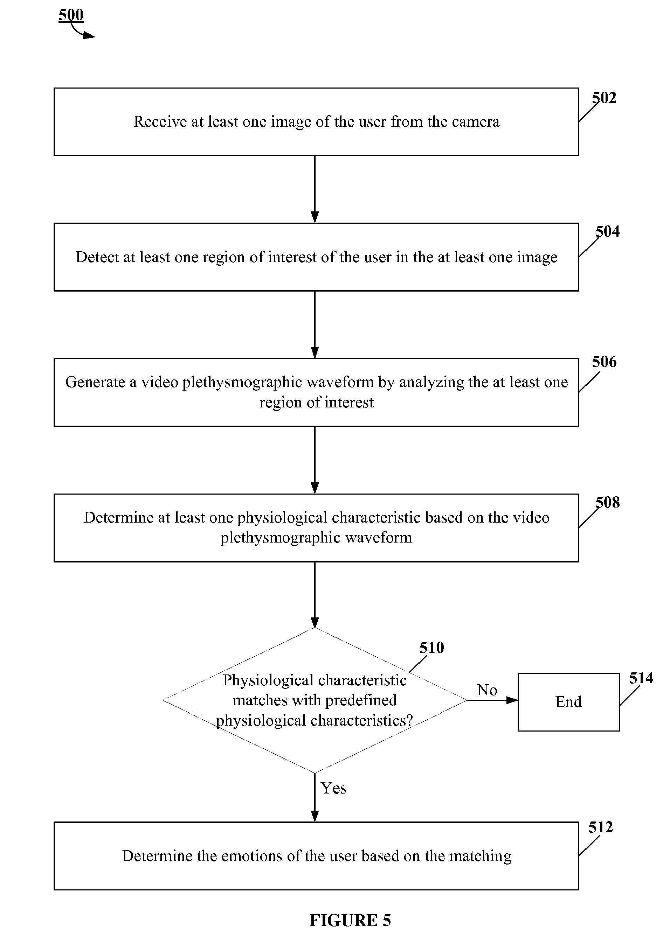

**Figure 5** shows a flowchart illustrating a method **500** for determining emotions of the user using the at least one camera **208** in accordance with some embodiments of the present disclosure.

As illustrated in **Figure 5****,** the method comprises one or more blocks for determining the emotions of the user. The method **500** may be described in the general context of computer executable instructions. Generally, computer executable instructions can include routines, programs, objects, components, data structures, procedures, modules, and functions, which perform particular functions or implement particular abstract data types.

The order in which the method **500** is described is not intended to be construed as a limitation, and any number of the described method blocks can be combined in any order to implement the method. Additionally, individual blocks may be deleted from the methods without departing from the scope of the subject matter described herein. Furthermore, the method can be implemented in any suitable hardware, software, firmware, or combination thereof.

At **block 502,** the at least one image of the user is received from the camera, and/or the one or more user devices and/or the one or more servers.

At **block 504,** the at least one region of interest of the user is detected. In an embodiment, uncovered body part of the user is detected as the at least one region of interest.

At **block 506,** the VPW of the corresponding at least one region of interest is generated by analyzing the corresponding at least one region of interest. In an embodiment, the video plethysmographic waveform is generated based on pixel variations of the image, corresponding to each of the at least one region of interest.

At **block 508,** the at least one physiological characteristic of the user is determined based on the VPW. In an embodiment, the at least one physiological characteristic comprises SPO2, the respiratory rate, and the heart rate of the user.

At **block 510,** the at least one physiological characteristic is compared with the predefined physiological characteristics defined for the emotions. If the at least one physiological characteristic matches with the predefined physiological characteristics then process goes to **block 512** via "Yes".

At **block 512,** the emotions of the user are determined based on the matching. If the at least one physiological characteristic does not match with the predefined physiological characteristics then process goes to **block 514** via "No" where the process is ended. In an embodiment, the feedback is received from the user/ any other persons to update the predefined physiological characteristics.

### Computer System

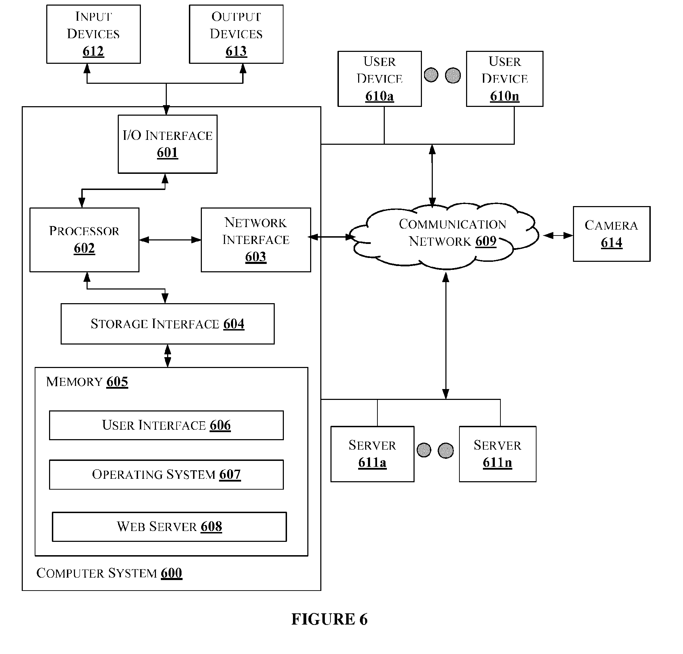

**Figure 6** illustrates a block diagram of an exemplary computer system **600** for implementing embodiments consistent with the present disclosure. In an embodiment, the computer system **600** is used to implement the emotion detection system **200.** The computer system **600** may comprise a central processing unit ("CPU" or "processor") **602.** The processor **602** may comprise at least one data processor for executing program components for executing system-generated video plethysmographic waveform for the corresponding region of interest. The processor **602** may include specialized processing units such as integrated system (bus) controllers, memory management control units, floating point units, graphics processing units, digital signal processing units, etc.

The processor **602** may be disposed in communication with one or more input/output (I/O) devices (not shown) via I/O interface **601.** The I/O interface **601** may employ communication protocols/methods such as, without limitation, audio, analog, digital, monoaural, RCA, stereo, IEEE-1394, serial bus, universal serial bus (USB), infrared, PS/2, BNC, coaxial, component, composite, digital visual interface (DVI), high-definition multimedia interface (HDMI), RF antennas, S-Video, VGA, IEEE 802.n /b/g/n/x, Bluetooth, cellular (e.g., code-division multiple access (CDMA), high-speed packet access (HSPA+), global system for mobile communications (GSM), long-term evolution (LTE), WiMax, or the like), etc.

Using the I/O interface **601,** the computer system **600** may communicate with one or more I/O devices. For example, the input device may be an antenna, keyboard, mouse, joystick, (infrared) remote control, camera, card reader, fax machine, dongle, biometric reader, microphone, touch screen, touchpad, trackball, stylus, scanner, storage device, transceiver, video device/source, etc. The output device may be a printer, fax machine, video display (e.g., cathode ray tube (CRT), liquid crystal display (LCD), light-emitting diode (LED), plasma, Plasma display panel (PDP), Organic light-emitting diode display (OLED) or the like), audio speaker, etc.

In some embodiments, the computer system **600** is connected to the one or more user devices **611a,,,.611n,** the one or more servers **610a,...,610n** and the camera **614** through a communication network **609.** The processor **602** may be disposed in communication with the communication network **609** via a network interface **603.** The network interface **603** may communicate with the communication network **609.** The network interface **603** may employ connection protocols including, without limitation, direct connect, Ethernet (e.g., twisted pair 10/100/1000 Base T), transmission control protocol/internet protocol (TCP/IP), token ring, IEEE 802.11a/b/g/n/x, etc. The communication network **609** may include, without limitation, a direct interconnection, local area network (LAN), wide area network (WAN), wireless network (e.g., using Wireless Application Protocol), the Internet, etc. Using the network interface **603** and the communication network **609,** the computer system **600** may communicate with the one or more user devices **611a,,,.611n,** the one or more servers **610a,...,610n** and the camera **614.** The network interface **603** may employ connection protocols include, but not limited to, direct connect, Ethernet (e.g., twisted pair 10/100/1000 Base T), transmission control protocol/internet protocol (TCP/IP), token ring, IEEE 802.11a/b/g/n/x, etc.

The communication network **609** includes, but is not limited to, a direct interconnection, an e-commerce network, a peer to peer (P2P) network, local area network (LAN), wide area network (WAN), wireless network (e.g., using Wireless Application Protocol), the Internet, Wi-Fi and such. The first network and the second network may either be a dedicated network or a shared network, which represents an association of the different types of networks that use a variety of protocols, for example, Hypertext Transfer Protocol (HTTP), Transmission Control Protocol/Internet Protocol (TCP/IP), Wireless Application Protocol (WAP), etc., to communicate with each other. Further, the first network and the second network may include a variety of network devices, including routers, bridges, servers, computing devices, storage devices, etc.

In some embodiments, the processor **602** may be disposed in communication with a memory **605** (e.g., RAM, ROM, etc. not shown in **figure 6****)** via a storage interface **604.** The storage interface **604** may connect to memory **605** including, without limitation, memory drives, removable disc drives, etc., employing connection protocols such as serial advanced technology attachment (SATA), Integrated Drive Electronics (IDE), IEEE-1394, Universal Serial Bus (USB), fiber channel, Small Computer Systems Interface (SCSI), etc. The memory drives may further include a drum, magnetic disc drive, magneto-optical drive, optical drive, Redundant Array of Independent Discs (RAID), solid-state memory devices, solid-state drives, etc.

The memory **605** may store a collection of program or database components, including, without limitation, user interface **606,** an operating system **607,** web server **608** etc. In some embodiments, computer system **600** may store user/application data **606,** such as the data, variables, records, etc. as described in this disclosure. Such databases may be implemented as fault-tolerant, relational, scalable, secure databases such as Oracle or Sybase.

The operating system **607** may facilitate resource management and operation of the computer system **600.** Examples of operating systems include, without limitation, Apple Macintosh OS X, Unix, Unix-like system distributions (e.g., Berkeley Software Distribution (BSD), FreeBSD, NetBSD, OpenBSD, etc.), Linux distributions (e.g., Red Hat, Ubuntu, Kubuntu, etc.), IBM OS/2, Microsoft Windows (XP, Vista/7/8, etc.), Apple iOS, Google Android, Blackberry OS, or the like.

In some embodiments, the computer system **600** may implement a web browser **607** stored program component. The web browser **608** may be a hypertext viewing application, such as Microsoft Internet Explorer, Google Chrome, Mozilla Firefox, Apple Safari, etc. Secure web browsing may be provided using Secure Hypertext Transport Protocol (HTTPS), Secure Sockets Layer (SSL), Transport Layer Security (TLS), etc. Web browsers **608** may utilize facilities such as AJAX, DHTML, Adobe Flash, JavaScript, Java, Application Programming Interfaces (APIs), etc. In some embodiments, the computer system **600** may implement a mail server stored program component. The mail server may be an Internet mail server such as Microsoft Exchange, or the like. The mail server may utilize facilities such as ASP, ActiveX, ANSI C++/C#, Microsoft .NET, CGI scripts, Java, JavaScript, PERL, PHP, Python, WebObjects, etc. The mail server may utilize communication protocols such as Internet Message Access Protocol (IMAP), Messaging Application Programming Interface (MAPI), Microsoft Exchange, Post Office Protocol (POP), Simple Mail Transfer Protocol (SMTP), or the like. In some embodiments, the computer system **600** may implement a mail client stored program component. The mail client may be a mail viewing application, such as Apple Mail, Microsoft Entourage, Microsoft Outlook, Mozilla Thunderbird, etc.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include Random Access Memory (RAM), Read-Only Memory (ROM), volatile memory, nonvolatile memory, hard drives, CD ROMs, DVDs, flash drives, disks, and any other known physical storage media.

Advantages of the embodiment of the present disclosure are illustrated herein.

Embodiments of the present disclosure are capable of determining emotions of the user using just a camera, that is, from image clips or video clips of the user. In such a way, usage of wearable devices is eliminated, user contact is eliminated and also the application of the present disclosure is cost effective.

Embodiments of the present disclosure reduce measuring physiological characteristics erroneously by generating video plethysmographic waveforms which actually indicates the actual emotions of the user.

Embodiments of the present disclosure provide dynamic technique of determining the emotions of the user using the images of the user in real-time and the video plethysmographic waveforms corresponding to the region of interest of the user from the image.

Embodiments of the present disclosure can determine the actual hidden emotions of the user without reading the facial expressions.

The described operations may be implemented as a method, system or article of manufacture using standard programming and/or engineering techniques to produce software, firmware, hardware, or any combination thereof. The described operations may be implemented as code maintained in a "non-transitory computer readable medium", where a processor may read and execute the code from the computer readable medium. The processor is at least one of a microprocessor and a processor capable of processing and executing the queries. A non-transitory computer readable medium may comprise media such as magnetic storage medium (e.g., hard disk drives, floppy disks, tape, etc.), optical storage (CD-ROMs, DVDs, optical disks, etc.), volatile and nonvolatile memory devices (e.g., EEPROMs, ROMs, PROMs, RAMs, DRAMs, SRAMs, Flash Memory, firmware, programmable logic, etc.), etc. Further, non-transitory computer-readable media comprise all computer-readable media except for a transitory. The code implementing the described operations may further be implemented in hardware logic (e.g., an integrated circuit chip, Programmable Gate Array (PGA), Application Specific Integrated Circuit (ASIC), etc.).

Still further, the code implementing the described operations may be implemented in "transmission signals", where transmission signals may propagate through space or through a transmission media, such as an optical fiber, copper wire, etc. The transmission signals in which the code or logic is encoded may further comprise a wireless signal, satellite transmission, radio waves, infrared signals, Bluetooth, etc. The transmission signals in which the code or logic is encoded is capable of being transmitted by a transmitting station and received by a receiving station, where the code or logic encoded in the transmission signal may be decoded and stored in hardware or a non-transitory computer readable medium at the receiving and transmitting stations or devices. An "article of manufacture" comprises non-transitory computer readable medium, hardware logic, and/or transmission signals in which code may be implemented. A device in which the code implementing the described embodiments of operations is encoded may comprise a computer readable medium or hardware logic. Of course, those skilled in the art will recognize that many modifications may be made to this configuration without departing from the scope of the invention, and that the article of manufacture may comprise suitable information bearing medium known in the art.

The terms "an embodiment", "embodiment", "embodiments", "the embodiment", "the embodiments", "one or more embodiments", "some embodiments", and "one embodiment" mean "one or more (but not all) embodiments of the invention(s)" unless expressly specified otherwise.

The terms "including", "comprising", "having" and variations thereof mean "including but not limited to", unless expressly specified otherwise.

The enumerated listing of items does not imply that any or all of the items are mutually exclusive, unless expressly specified otherwise.

The terms "a", "an" and "the" mean "one or more", unless expressly specified otherwise.

A description of an embodiment with several components in communication with each other does not imply that all such components are required. On the contrary a variety of optional components are described to illustrate the wide variety of possible embodiments of the invention.

When a single device or article is described herein, it will be readily apparent that more than one device/article (whether or not they cooperate) may be used in place of a single device/article. Similarly, where more than one device or article is described herein (whether or not they cooperate), it will be readily apparent that a single device/article may be used in place of the more than one device or article or a different number of devices/articles may be used instead of the shown number of devices or programs. The functionality and/or the features of a device may be alternatively embodied by one or more other devices which are not explicitly described as having such functionality/features. Thus, other embodiments of the invention need not include the device itself.

The illustrated operations of **Figure 5** show certain events occurring in a certain order. In alternative embodiments, certain operations may be performed in a different order, modified or removed. Moreover, steps may be added to the above described logic and still conform to the described embodiments. Further, operations described herein may occur sequentially or certain operations may be processed in parallel. Yet further, operations may be performed by a single processing unit or by distributed processing units.

Finally, the language used in the specification has been principally selected for readability and instructional purposes, and it may not have been selected to delineate or circumscribe the inventive subject matter. It is therefore intended that the scope of the invention be limited not by this detailed description, but rather by any claims that issue on an application based here on. Accordingly, the disclosure of the embodiments of the invention is intended to be illustrative, but not limiting, of the scope of the invention, which is set forth in the following claims.

While various aspects and embodiments have been disclosed herein, other aspects and embodiments will be apparent to those skilled in the art. The various aspects and embodiments disclosed herein are for purposes of illustration and are not intended to be limiting, with the true scope being indicated by the appended claims.

## Claims

1. A processor implemented method for determining emotions of a user using a camera, the method comprising:
receiving at least one images of the user from the camera;
detecting at least one region of interest of the user in the at least one image;
generating a video plethysmographic waveform by analyzing the at least one region of interest;
determining at least one physiological characteristic based on the video plethysmographic waveform; and
determining the emotions of the user by comparing the at least one physiological characteristic with one or more predefined physiological characteristics defined for each emotion.

2. The method as claimed in claim 1, wherein the region of interest comprises uncovered body parts of the user.

3. The method as claimed in claim 1 or claim 2, wherein the at least one physiological characteristic comprises at least one of a peripheral capillary oxygen saturation (SPO2), a respiratory rate, and a heart rate of the user.

4. The method as claimed in any preceding claim, comprising generating the video plethysmographic waveform based on pixel variations of the image, corresponding to each of the at least one region of interest.

5. The method as claimed in claim 4 comprising determining the emotion of the user after receiving more than one of the images for a predetermined time period.

6. The method as claimed in claim 5 wherein the predetermined time period is 30 seconds.

7. The method as claimed in any preceding claim wherein analyzing the at least one region of interest comprises generating a color histogram from the more than one of the images and generating a trace of one or more of the colors of the histogram, and then generating the video plethysmographic waveform based on the or each trace.

8. The method as claimed in claim 7 wherein the color histogram includes a red channel, and/or a blue channel, and/or a green channel.

9. The method as claimed in any preceding claim, wherein the emotions of the user is one of happiness, sadness, fear, anger, surprise and disgust.

10. The method as claimed in any preceding claim, wherein the predefined physiological characteristics defined for the each emotion are updated based on a feedback received on the emotions determined for the user.

11. An emotion detection system for determining emotions of a user using a camera comprising:
a processor;
a memory communicatively coupled to the processor, wherein the memory stores processor-executable instructions, which, on execution, cause the processor to perform the processor implemented method as claimed in any preceding claim.

12. A non-transitory computer readable medium including instructions stored thereon that when processed by a processor cause an emotion detection system for determining emotions of a user using a camera by performing the method as claimed in any of claims 1 to 10.
